# EUROPÄISCHE PATENTANMELDUNG

(11) **EP 4 029 424 A1**
(43) Veröffentlichungstag der Anmeldung: **20.07.2022**
(21) Anmeldenummer: 21151683.6
(22) Anmeldetag: 14.01.2021
(51) Int. Cl.: A61B 1/00, A61B 1/06, G02B 23/24, H01L 33/62

(54) **ULTRA-MINIATURISIERTE LICHTEMITTIERENDE EINHEIT FÜR EIN MEDIZINISCH-ENDOSKOPISCHES INSTRUMENT**

(71) Anmelder: Richard Wolf GmbH, 75438 Knittlingen (DE)
(72) Erfinder: Langen, Sebastian, 75438 Knittlingen (DE)
(74) Vertreter: Patentanwälte Vollmann Hemmer Lindfeld Partnerschaft mbB

(57) **Zusammenfassung**

Die vorliegende Offenbarung betrifft eine ultra-miniaturisierte lichtemittierende Einheit (1) für ein medizinisch-endoskopisches Instrument, wobei die lichtemittierende Einheit (1) eine Hauptabstrahlungsrichtung (H) definiert, aufweisend
- ein sich im Wesentlichen orthogonal zur Hauptabstrahlungsrichtung (H) flächig erstreckendes Halbleiterelement (3) mit einer in Hauptabstrahlungsrichtung (H) gewandten Lichtabstrahlseite (9) und einer der Hauptabstrahlungsrichtung (H) abgewandten Kontaktseite (21),
- ein sich im Wesentlichen in Hauptabstrahlungsrichtung (H) erstreckendes Koaxialkabel mit einem Innenleiter und einem Außenleiter zur Stromversorgung der Einheit (1) und
- ein sich im Wesentlichen in Hauptabstrahlungsrichtung (H) erstreckendes Isolierelement (5),
wobei das Isolierelement (5) einen ersten Abschnitt (27) und einen am Halbleiterelement (3) lateral vorbeigeführten zweiten Abschnitt (29) aufweist, sodass ein Teil (31) des zweiten Abschnitts (29) in Hauptabstrahlungsrichtung (H) über die Lichtabstrahlseite (9) des Halbleiterelements (3) hinausragt,
wobei ein Leiterelement (25) im Isolierelement (5) eingebettet ist und sich vom ersten Abschnitt (27) des Isolierelements (5) durch den zweiten Abschnitt (29) des Isolierelements (5) erstreckt, wobei ein erster Kontaktbereich (49) des Leiterelements (25) im ersten Abschnitt (27) des Isolierelements (5) mit dem Außenleiter (13) elektrisch leitend kontaktiert ist und ein zweiter Kontaktbereich (53) des Leiterelements (25) im zweiten Abschnitt (29) des Isolierelements (5) mittels einer Kehllötung (52) mit der Lichtabstrahlseite (9) des Halbleiterelements (3) elektrisch leitend kontaktiert ist,
und wobei die Kontaktseite (21) des Halbleiterelements (3) mit dem Innenleiter elektrisch kontaktiert ist.

## Beschreibung

Die vorliegende Offenbarung betrifft eine ultra-miniaturisierte lichtemittierende Einheit für ein medizinisch-endoskopisches Instrument und ein medizinisch-endoskopisches Instrument mit solch einer ultra-miniaturisierten lichtemittierenden Einheit.

Es ist beispielweise aus der DE 10 2009 049 683 B4 bekannt, eine lichtemittierende Diode (LED) oder mehr an medizinisch-endoskopischen Instrumenten vorzusehen, um damit das Innere eines menschlichen oder tierischen Körpers von innen beleuchten oder mit Licht bestrahlen zu können. Um allerdings das Einführen eines medizinisch-endoskopischen Instruments möglichst minimal-invasiv zu gestalten, ist es ein dauerhaftes Bestreben, die länglichen distalen Einführabschnitte solcher Instrumente möglichst schmal und fein auszugestalten. Eine Grenze für diese Miniaturisierung stellen bislang verwendete LEDs dar, deren laterale flächige Ausdehnung meist mehr als 0,5 mm² beträgt. Ein Grund dafür ist eine über den eigentlichen LED-Chip hinausgehende flächige Ausdehnung eines Basiselements zur Anbringung eines Bonddrahtes, der den Pluspol bzw. die Anode einer Versorgungsgleichspannung mit der p-dotierten Lichtabstrahlseite des LED-Chips elektrisch verbindet.

Aus der DE 10 2008 202 241 A1 ist eine ultra-miniaturisierte lichtemittierende Einheit bekannt mit einem L-förmig ein Halbleiterelement einer LED umgreifenden Isolierelement in Form einer flexiblen Leiterplatte. Bei der Umsetzung dieser Lösung haben sich allerdings Probleme beim Falten bzw. Biegen der flexiblen Leiterplatte ergeben.

Die vorliegende Offenbarung stellt dagegen eine lichtemittierende Einheit mit einer lateralen flächigen Ausdehnung bereit, die möglichst wenig über die laterale flächige Ausdehnung des eigentlichen LED-Chips hinausgeht und ohne Falten bzw. Biegen einer flexiblen Leiterplatte auskommt. Es wird somit erreicht, dass die lichtemittierende Einheit ultra-miniaturisiert werden kann, d. h. eine laterale flächige Ausdehnung von 0,5 mm² oder weniger hat. Dies verschiebt die Grenze für die Miniaturisierung von distalen Einführabschnitten medizinischendoskopischer Instrumente hin zu immer kleineren und damit weniger traumatischen Durchmessern.

Gemäß einem ersten Aspekt der vorliegenden Offenbarung wird eine ultra-miniaturisierte lichtemittierende Einheit für ein medizinisch-endoskopisches Instrument bereitgestellt, wobei die lichtemittierende Einheit eine Hauptabstrahlungsrichtung definiert, und ein sich im Wesentlichen orthogonal zur Hauptabstrahlungsrichtung flächig erstreckendes Halbleiterelement mit einer in Hauptabstrahlungsrichtung gewandten Lichtabstrahlseite und einer der Hauptabstrahlungsrichtung abgewandten Kontaktseite, ein sich im Wesentlichen in Hauptabstrahlungsrichtung erstreckendes Koaxialkabel mit einem Innenleiter und einem Außenleiter zur Stromversorgung der Einheit, und ein Isolierelement aufweist. Das Isolierelement weist dabei einen ersten Abschnitt und einen am Halbleiterelement lateral vorbeigeführten zweiten Abschnitt auf, sodass ein Teil des L-förmigen zweiten Abschnitts in Hauptabstrahlungsrichtung über die Lichtabstrahlseite des Halbleiterelements hinausragt. Ein Leiterelement ist dabei im Isolierelement eingebettet und erstreckt sich vom ersten Abschnitt des Isolierelements durch den zweiten Abschnitt des Isolierelements, wobei ein erster Kontaktbereich des Leiterelements im ersten Abschnitt des Isolierelements mit dem Außenleiter elektrisch leitend kontaktiert ist und ein zweiter Kontaktbereich des Leiterelements im zweiten Abschnitt des Isolierelements mittels einer Kehllötung mit der Lichtabstrahlseite des Halbleiterelements elektrisch kontaktiert ist, und wobei die Kontaktseite des Halbleiterelements mit dem Innenleiter elektrisch leitend kontaktiert ist.

Mit solch einem Aufbau wird die laterale Ausdehnung der Einheit minimiert, da die Kehllötung einen Bonddraht ersetzt und daher keine über das Halbleiterelement hinausgehende flächige Ausdehnung des Basiselements benötigt wird. Das Halbleiterelement kann hierbei einen LED-Chip mit einer lateralen Flächenausdehnung von 0,25 mm² oder weniger darstellen, dessen Lichtabstrahlseite vorzugsweise p-dotiert sein kann. Die Kontaktseite des Halbleiterelements kann vorzugsweise n-dotiert und mit einer leitenden und reflektierenden Metallschicht bedampft sein. Die Hauptabstrahlungsrichtung sei hier als die Raumrichtung mit maximaler Lichtabstrahlungsintensität definiert, was in diesem Fall der Normalen auf die Lichtabstrahlseite des Halbleiterelements entspricht. Das Halbleiterelement ist vorzugsweise Gallium-basiert und kann beispielsweise Indiumgalliumnitrid (InGaN oder InₓGa₁₋ₓN) aufweisen.

Der Außenleiter kann hier an einen Pluspol bzw. die Anode einer Gleichspannungsversorgungsspannung und der Innenleiter an einen entsprechenden Minuspol bzw. die Kathode angeschlossen sein. Das Halbleiterelement als Diode wird damit in Stromdurchlassrichtung, die im Wesentlichen der Hauptabstrahlungsrichtung entspricht, mit Strom durchflossen, wobei sich im Bereich des Übergangs vom n-dotierten Bereich zum p-dotierten Bereich innerhalb des Halbleiterelements Photonen bilden und als Licht abgestrahlt werden. Entgegen der Hauptabstrahlungsrichtung abgestrahltes Licht kann dabei durch eine reflektierende Metallschicht auf der Kontaktseite des Halbleiterelements im Wesentlichen in die Hauptabstrahlungsrichtung reflektiert werden.

In einer optionalen Ausführungsform kann der Außenleiter mit dem ersten Kontaktbereich des Leiterelements im ersten Abschnitt des Isolierelements verlötet sein. Dies dient zum einen dem elektrischen Kontakt und zum anderen der mechanischen Befestigung des Isolierelements am Koaxialkabel.

Optional kann der zweite Abschnitt des Isolierelements am Halbleiterelement lateral anliegend an diesem in Hauptabstrahlungsrichtung vorbeigeführt sein. Damit wird die für Kontaktierung der Lichtabstrahlseite benötigte laterale Ausdehnung auf die Dicke des Isolierelements minimiert.

Optional kann das Isolierelement samt eingebettetem Leiterelement flexibel oder starr sein. Beispielsweise kann das Isolierelement samt eingebettetem Leiterelement als starre oder flexible Leiterplatte ausgestaltet sein. Das Isolierelement kann beispielsweise eine Polyamid-Folie aufweisen, in die eine Leiterbahn eingebettet ist. Die Leiterplatte inklusive eingebettetem Leiterelement kann sehr dünn ausgestaltet sein, etwa mit einer Dicke von 25 µm oder weniger. Dadurch kann der am Halbleiterelement lateral vorbeigeführte zweite Abschnitt nur eine vernachlässigbar kleine laterale Ausdehnung einnehmen. Die Leiterplatte kann derart ausgestaltet sein, dass der zweite Abschnitt im Wesentlichen senkrecht zur Lichtabstrahlseite über eine Kante der Lichtabstrahlseite teilweise in Hauptabstrahlungsrichtung hinausragt. Eine flexible Leiterplatte hat dabei den Vorteil, dass sie außerordentlich resilient gegen Verformungen während des Zusammenbaus der Einheit ist und nicht dabei bricht oder abknickt.

Optional kann der zweite Abschnitt des Isolierelements in Hauptabstrahlungsrichtung zumindest um eine Dicke des Halbleiterelements in Hauptabstrahlungsrichtung über die Lichtabstrahlseite des Halbleiterelements hinausragen. Die Kehllötung kann dann lateral mit dem Überstand und proximalseitig mit der Lichtabstrahlseite in Kontakt stehen.

Optional kann sich das Isolierelement in einer zur Hauptabstrahlungsrichtung parallel verlaufenden Ebene planar erstrecken und eine zur Hauptabstrahlungsrichtung quer verlaufende Breite aufweist, wobei die Breite des in Hauptabstrahlungsrichtung über die Lichtabstrahlseite des Halbleiterelements hinausragenden Teils des zweiten Abschnitts des Isolierelements geringer ist als die Breite des ersten Abschnitts des Isolierelements. Die Breite des ersten Abschnitts des Isolierelements entspricht vorzugsweise in etwas der Breite des Halbleiterelements oder weniger. Auch der Durchmesser des Koaxialkabels, ggf. inklusive Außenisolierung, beträgt in etwas der Breite des Halbleiterelements oder weniger. Dadurch ist die laterale Ausdehnung der gesamten Einheit im Wesentlichen durch die lateralen Abmessungen des Halbleiterelements bestimmt. Der in Hauptabstrahlungsrichtung über die Lichtabstrahlseite des Halbleiterelements hinausragende Teil des zweiten Abschnitts des Isolierelements ist vorzugsweise so schmal wie möglich, um gerade einen hinreichend großen zweiten Kontaktbereich für die Kehllötung zur Verfügung zu stellen. Die Kehllötung erstreckt sich vorzugsweise im Wesentlichen über die gesamte Breite des in Hauptabstrahlungsrichtung über die Lichtabstrahlseite des Halbleiterelements hinausragenden Teils des zweiten Abschnitts des Isolierelements.

Optional kann die Breite des in Hauptabstrahlungsrichtung über die Lichtabstrahlseite des Halbleiterelements hinausragenden Teils des zweiten Abschnitts des Isolierelements weniger als die Hälfte oder ein Drittel der Breite des ersten Abschnitts des Isolierelements betragen. Die Kehllötung ist vorzugsweise so klein wie möglich, sodass durch diese nur höchstens 1/9 der Lichtabstrahlseite des Halbleiterelements abgedeckt werden.

Optional kann das sich der in Hauptabstrahlungsrichtung über die Lichtabstrahlseite des Halbleiterelements hinausragende Teil des zweiten Abschnitts des Isolierelements im Wesentlichen senkrecht zur Lichtabstrahlseite des Halbleiterelements erstrecken. Die Kehllötung ist dann eine besonders stabile 90°-Kehllötung.

Optional kann der Innenleiter des Koaxialkabels lateral am Isolierelement befestigt sein, wobei der Innenleiter vom Leiterelement im Isolierelement elektrisch isoliert ist. Vorzugsweise ist der Innenleiter mit einem nichtleitenden Kleber mit dem Isolierelement verklebt, vorzugsweise in Hauptabstrahlrichtung zwischen dem ersten Kontaktbereich und dem Halbleiterelement. Dadurch wird das Isolierelement zusätzlich zur Verlötung der Außenleiters mit dem ersten Kontaktbereich mechanisch am Koaxialkabel fixiert. Dies dient auch der Sicherung des elektrischen Kontakts zwischen dem distalen Stirnende des Innenleiters mit der Kontaktseite des Halbleiterelements. Der Innenleiter ist vorzugsweise möglichst weit distal am Isolierelement lateral befestigt.

Optional kann das Isolierelement eine erste Kontaktöffnung für den ersten Kontaktbereich des Leiterelements und eine zweite Kontaktöffnung für den zweiten Kontaktbereich des Leiterelements aufweisen, wobei die erste Kontaktöffnung zum Außenleiter des Koaxialkabels hin geöffnet ist und die zweite Kontaktöffnung eine Ausnehmung in dem in Hauptabstrahlungsrichtung über die Lichtabstrahlseite des Halbleiterelements hinausragenden Teil des zweiten Abschnitts ist. Dadurch wird eine Kontaktierung des Leiterelements während des Zusammenbaus der Einheit besonders einfach und zuverlässig. Durch die Ausnehmung kann zum einen ein elektrischer Kontakt zwischen dem zweiten Kontaktbereich des Leiterelements und der Kehllötung hergestellt werden, und zum anderen der zweite Abschnitt des Isolierelements samt dem Leiterelement am Halbleiterelement zusätzlich mechanisch fixiert werden. Die Ausnehmung kann vollständig oder nur teilweise von dem in Hauptabstrahlungsrichtung über die Lichtabstrahlseite des Halbleiterelements hinausragenden Teil des zweiten Abschnitts lateral umgeben sein. Das bedeutet, dass die Ausnehmung ein Loch und/oder eine Aussparung sein kann.

Optional kann der zweite Kontaktbereich des Leiterelements in die zweite Kontaktöffnung des Isolierelements hineinragen, wobei die Kehllötung die zweite Kontaktöffnung vollständig füllt und den elektrischen Kontakt zwischen dem zweiten Kontaktbereich des Leiterelements und der Lichtabstrahlseite des Halbleiterelements herstellt.

Gemäß einem zweiten Aspekt der vorliegenden Offenbarung wird ein medizinisch-endoskopisches Instrument bereitgestellt mit einem distalen länglichen Einführabschnitt zum minimal-invasiven Einführen in einen menschlichen oder tierischen Körper, wobei der Einführabschnitt mindestens eine zuvor beschriebene ultra-miniaturisierte lichtemittierende Einheit aufweist, um damit den Körper von innen beleuchten und/oder mit Licht bestrahlen zu können.

Optional kann dabei zumindest eine der mindestens einen ultra-miniaturisierten lichtemittierenden Einheit an einer distalen Stirnseite des Einführabschnitts angeordnet sein.

Die Offenbarung ist nachfolgend anhand von einem in den Zeichnungen dargestellten Ausführungsbeispiel näher erläutert. Es zeigen:
Fig. 1 eine perspektivische Ansicht auf eine beispielhafte Ausführungsform der hierin offenbarten ultra-miniaturisierten lichtemittierenden Einheit; und
Fig. 2 eine perspektivische Seitenansicht auf eine beispielhafte Ausführungsform eines distalen Einführabschnitts des hierin offenbarten medizinisch-endoskopischen Instruments.

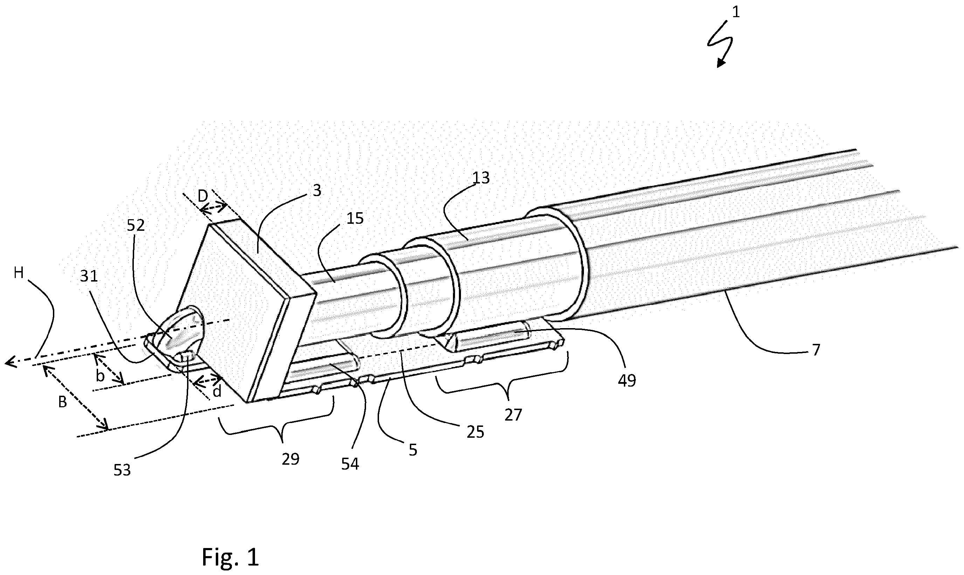

Figur 1 zeigt eine ultra-miniaturisierte lichtemittierende Einheit 1 mit einem flächigen Halbleiterelement 3, einem planaren Isolierelement 5 und einem Koaxialkabel 7 zur Stromversorgung der Einheit 1, wobei sich das Isolierelement 5 lateral am Koaxialkabel 7 and lateral am Halbleiterelement 3 entlang erstreckt. Die Einheit 1 definiert eine Hauptabstrahlungsrichtung H, die sich im Wesentlichen senkrecht zur lateralen Ausdehnungsfläche des Halbleiterelements 3 erstreckt. Eine in Hauptabstrahlungsrichtung H weisende Seite 9 des Halbleiterelements 3 kann damit auch als Lichtabstrahlseite 9 bezeichnet werden. Zum besseren Verständnis der Fig. 1 kann hier auch von einer distalen Lichtabstrahlseite 9 gesprochen werden, wenngleich die Einheit 1 und damit die Hauptabstrahlungsrichtung H in jedwede Raumrichtung ausgerichtet sein kann. Im Folgenden soll "distal" bzw. "distalwärts" oder "distalseitig" in Richtung der Hauptabstrahlungsrichtung H verstanden werden und "proximal" bzw. "proximalwärts" oder "proximalseitig" entgegen der Hauptabstrahlungsrichtung H. In diesem Sinne ist das Koaxialkabel 7 der Einheit 1 von proximal an das Halbleiterelement 3 angeschlossen.

Das Koaxialkabel 7 weist einen Außenleiter 13 und einen Innenleiter 15 auf, wobei der Außenleiter 13 als Pluspol bzw. Anode einer Gleichspannungsversorgung und der Innenleiter 15 als Minuspol bzw. Kathode der Gleichspannungsversorgung fungiert. Der Außenleiter 13 und der Innenleiter 15 sind voneinander isoliert durch das Koaxialkabel 7 geführt.

Das Halbleiterelement 3 mit einer Dicke D Hauptabstrahlungsrichtung H in dient als eigentlicher LED-Chip und Leuchtkörper. Es ist in dieser Ausführungsform quaderförmig mit im Wesentlichen quadratischer Lichtabstrahlseite 9 und im distalen Bereich p-dotiert und im proximalen Bereich n-dotiert. Eine Kontaktseite 21 des Halbleiterelements 3, die hier der proximalen Seite 21 des Halbleiterelements 3 entspricht, ist hier mit einer elektrisch leitenden und lichtreflektierenden Metallschicht bedampft. Dadurch wird innerhalb des Halbleiterelements 3 proximalwärts abgestrahltes Licht distalwärts reflektiert. Um das Halbleiterelement 3 zum Leuchten zu bringen, muss ein Strom am Übergang vom p-dotierten distalen Bereich in den n-dotierten proximalen Bereich des Halbleiterelements 3 fließen. In einer Ecke auf der Lichtabstrahlseite 9 des Halbleiterelements 3 ist dazu eine Kontaktfläche auf der Lichtabstrahlseite 9 angeordnet.

Das Isolierelement 5 ist hier als flexible oder starre Leiterplatte ausgestaltet mit einem eingebetteten Leiterelement 25 (gestrichelt dargestellt), das zur Kontaktierung mit der Kontaktfläche auf der Ecke der Lichtabstrahlseite 9 des Halbleiterelements 3 ausgestaltet ist. Das Isolierelement 5 weist in einen proximalen ersten Abschnitt 27 und einen distalen zweiten Abschnitt 29 auf. Ein schmalerer Teil 31 des zweiten Abschnitts 29 ragt dabei distal über die Lichtabstrahlseite 9 des Halbleiterelements 3 hinaus. Das Leiterelement 25 weist einen ersten Kontaktbereich 49 im ersten Abschnitt 27 und einen zweiten Kontaktbereich 53 in dem über die Lichtabstrahlseite 9 des Halbleiterelements 3 hinausragenden Teil 31 des zweiten Abschnitts 29 auf. Der erste Kontaktbereich 49 ist mit dem Außenleiter 13 elektrisch kontaktiert. Der zweite Kontaktbereich 53 ist mittels einer Kehllötung 52 mit der Ecke der Lichtabstrahlseite 9 des Halbleiterelements 3 elektrisch kontaktiert. Der Innenleiter 15 ist vom Leiterelement 25 elektrisch isoliert und an einer Verbindungsstelle 54 lateral mit dem Isolierelement 5 verbunden, vorzugsweise verklebt.

Das in das Isolierelement 5 eingebettete Leiterelement 25 erstreckt sich vom ersten Abschnitt 27 des Isolierelements 5 durch den zweiten Abschnitt 29 des Isolierelements 5. Das Leiterelement 25 ist hier im Wesentlichen planar ausgestaltet mit einer Breite B, die im Wesentlichen der Breite des Halbleiterelements 3 entspricht, oder ein wenig kleiner ist. Der distal über die Lichtabstrahlseite 9 des Halbleiterelements 3 hinausragende Teil 31 des zweiten Abschnitts 29 hat eine Breite b, die wesentlich geringer ist als die Breite B desersten Abschnitts 27, d.h. b < B. Hier beträgt die Breits b sogar weniger als die Hälfte der Breite B, d.h. b < B/2. Der Teil 31 hat in Hauptabstrahlungsrichtung H eine Länge d, die mindestens der Dicke D des Halbleiterelements 3 entspricht.

Um die elektrische Kontaktierung des eingebetteten Leiterelements 25 zu ermöglichen, weist das Isolierelement 5 eine erste Kontaktöffnung für den ersten Kontaktbereich 49 des Leiterelements 25 und eine zweite Kontaktöffnung für den zweiten Kontaktbereich 53 des Leiterelements 25 auf. Die erste Kontaktöffnung ist dabei zum Außenleiter 13 hin geöffnet, und die zweite Kontaktöffnung ist zur Kehllötung 52 hin geöffnet. Der erste Kontaktbereich 49 des Leiterelements 25 steht mit dem Außenleiter 13, d.h. dem Pluspol bzw. der Anode, des Koaxialkabels 7 in elektrischem Kontakt, vorzugsweise über eine Verlötung oder Silberleitkleberverbindung. Der zweite Kontaktbereich 53 des Leiterelements 25 steht mittels der Kehllötung 52 mit der Lichtabstrahlseite 9 des Halbleiterelements 3 in elektrischem Kontakt.

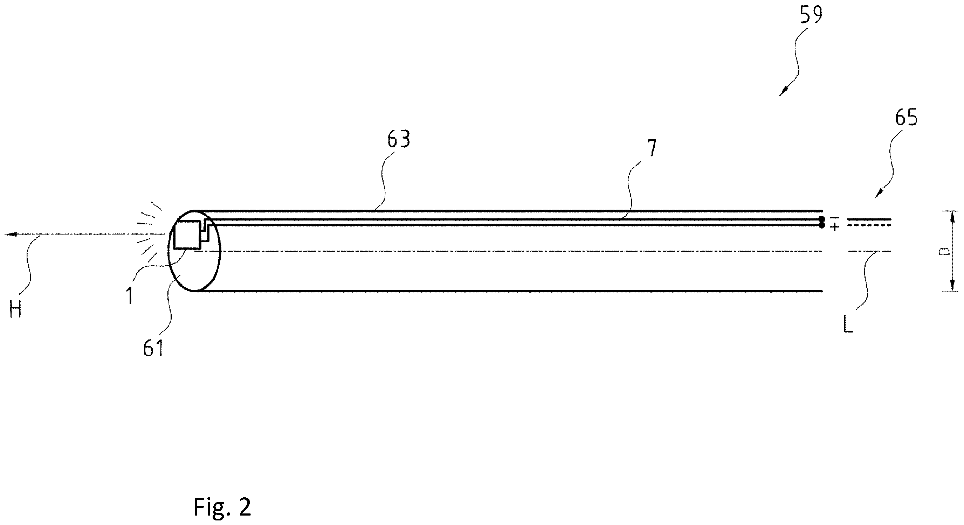

Die laterale Ausdehnung der Einheit 1 quer zur Hauptabstrahlungsrichtung H ragt hier nur vernachlässigbar gering über die laterale Ausdehnung des LED-Chips 3 selbst hinaus. Bei einem Einbau an der Stirnseite 61 eines wie in Fig. 2 gezeigten medizinisch-endoskopischen Instruments 59 nimmt die Einheit 1 damit weniger Bauraum ein, sodass der Durchmesser D eines distalen Einführabschnitts 63 des minimal-invasiven Instruments 59 verringert werden kann oder dort mehr Platz für andere Funktionen zur Verfügung steht.

Der zweite Abschnitt 29 des Isolierelements 5 ragt mit dem schmalen Teil 31 distal über die Lichtabstrahlseite 9 des Halbleiterelements 3 hinaus. Dabei liegt der zweite Abschnitt 29 des Isolierelements 5 lateral am Halbleiterelement 3 an. Zur mechanischen Stabilisierung in dieser Position ist das Koaxialkabel 7 zwischen dem ersten Abschnitt 27 des Isolierelements 5 und dem Halbleiterelement 3 mit dem Isolierelement 5 an der Verbindungsstelle 54 verklebt.

In Figur 2 ist gezeigt wie die LED-Einheit 1 gemäß der zuvor beschriebenen Ausführungsform an der Stirnseite 61 angeordnet ist, sodass die Hauptabstrahlungsrichtung H im Wesentlichen parallel zur Längsachse L des Einführabschnitts 63 verläuft. Der Einführabschnitt 63 kann dabei gerade, flexibel, gekrümmt oder abknickbar ausgestaltet sein. Über die Länge des Einführabschnitts 63 kann eine proximale Gleichspannungsquelle 65 über das Koaxialkabel 7 zur LED-Einheit 1 geführt sein. Alternativ oder zusätzlich zu einer Anordnung an der Stirnseite 61 können ein oder mehrere LED-Einheiten an einer lateralen Seite des Einführabschnitts 63 angeordnet sein, um einen umgebenden Hohlraum im Körper eines Menschen oder Tieres auszuleuchten oder mit Licht zu bestrahlen.

Die nummerierten Bezeichnungen der Bauteile oder Bewegungsrichtungen als "erste", "zweite", "dritte" usw. sind hierin rein willkürlich zur Unterscheidung der Bauteile oder Bewegungsrichtungen untereinander gewählt und können beliebig anders gewählt werden. Es ist damit kein Bedeutungsrang verbunden. Eine Bezeichnung eines Bauteils oder technischen Merkmals als "erstes" soll nicht dahingehend missverstanden werden, dass es ein zweites Bauteil oder technisches Merkmal dieser Art geben muss. Außerdem können etwaige Verfahrensschritte, soweit nicht explizit anders erläutert oder zwingend erforderlich, in beliebiger Reihenfolge und/oder zeitlich teilweise oder ganz überlappend durchgeführt werden.

Äquivalente Ausführungsformen der hierin beschriebenen Parameter, Bauteile oder Funktionen, die in Anbetracht dieser Beschreibung einer fachlich versierten Person als offensichtlich erscheinen, seien hierin so erfasst als wären sie explizit beschrieben. Entsprechend soll der Schutzbereich der Ansprüche solche äquivalente Ausführungsformen umfassen. Als optional, vorteilhaft, bevorzugt, erwünscht oder ähnlich bezeichnete "kann"-Merkmale sind als optional zu verstehen und nicht als schutzbereichsbeschränkend.

Die beschriebenen Ausführungsformen sind als illustrative Beispiele zu verstehen und stellen keine abschließende Liste von möglichen Ausführungsformen dar. Jedes Merkmal, das im Rahmen einer Ausführungsform offenbart wurde, kann allein oder in Kombination mit einem oder mehreren anderen Merkmalen verwendet werden, unabhängig davon, in welcher Ausführungsform die Merkmale jeweils beschrieben wurden. Während mindestens ein Ausführungsbeispiel hierin beschrieben und gezeigt ist, seien Abwandlungen und alternative Ausführungsformen, die einer fachmännisch versierten Person in Anbetracht dieser Beschreibung als offensichtlich erscheinen, vom Schutzbereich dieser Offenbarung mit erfasst. Im Übrigen soll hierin weder der Begriff "aufweisen" zusätzliche andere Merkmale oder Verfahrensschritte ausschließen noch soll "ein" oder "eine" eine Mehrzahl ausschließen.

## Patentansprüche

1. Ultra-miniaturisierte lichtemittierende Einheit (1) für ein medizinisch-endoskopisches Instrument, wobei die lichtemittierende Einheit (1) eine Hauptabstrahlungsrichtung (H) definiert, aufweisend
- ein sich im Wesentlichen orthogonal zur Hauptabstrahlungsrichtung (H) flächig erstreckendes Halbleiterelement (3) mit einer in Hauptabstrahlungsrichtung (H) gewandten Lichtabstrahlseite (9) und einer der Hauptabstrahlungsrichtung (H) abgewandten Kontaktseite (21),
- ein sich im Wesentlichen in Hauptabstrahlungsrichtung (H) erstreckendes Koaxialkabel (7) mit einem Innenleiter (15) und einem Außenleiter (13) zur Stromversorgung der Einheit (1) und
- ein sich im Wesentlichen in Hauptabstrahlungsrichtung (H) erstreckendes Isolierelement (5),
wobei das Isolierelement (5) einen ersten Abschnitt (27) und einen am Halbleiterelement (3) lateral vorbeigeführten zweiten Abschnitt (29) aufweist, sodass ein Teil (31) des zweiten Abschnitts (29) in Hauptabstrahlungsrichtung (H) über die Lichtabstrahlseite (9) des Halbleiterelements (3) hinausragt,
wobei ein Leiterelement (25) im Isolierelement (5) eingebettet ist und sich vom ersten Abschnitt (27) des Isolierelements (5) durch den zweiten Abschnitt (29) des Isolierelements (5) erstreckt, wobei ein erster Kontaktbereich (49) des Leiterelements (25) im ersten Abschnitt (27) des Isolierelements (5) mit dem Außenleiter (13) elektrisch leitend kontaktiert ist und ein zweiter Kontaktbereich (53) des Leiterelements (25) im zweiten Abschnitt (29) des Isolierelements (5) mittels einer Kehllötung (52) mit der Lichtabstrahlseite (9) des Halbleiterelements (3) elektrisch leitend kontaktiert ist,
und wobei die Kontaktseite (21) des Halbleiterelements (3) mit dem Innenleiter (15) elektrisch leitend kontaktiert ist.

2. Ultra-miniaturisierte lichtemittierende Einheit (1) nach Anspruch 1, wobei der Außenleiter (13) mit dem ersten Kontaktbereich (49) des Leiterelements (25) im ersten Abschnitt (27) des Isolierelements (5) verlötet ist.

3. Ultra-miniaturisierte lichtemittierende Einheit (1) nach Anspruch 1 oder 2, wobei der zweite Abschnitt (29) des Isolierelements (5) am Halbleiterelement (3) lateral anliegend an diesem in Hauptabstrahlungsrichtung (H) vorbeigeführt ist.

4. Ultra-miniaturisierte lichtemittierende Einheit (1) nach einem der vorhergehenden Ansprüche, wobei der zweite Abschnitt (29) des Isolierelements (5) in Hauptabstrahlungsrichtung (H) zumindest um eine Dicke (D) des Halbleiterelements (3) in Hauptabstrahlungsrichtung (H) über die Lichtabstrahlseite (9) des Halbleiterelements (3) hinausragt.

5. Ultra-miniaturisierte lichtemittierende Einheit (1) nach einem der vorhergehenden Ansprüche, wobei sich das Isolierelement (5) in einer zur Hauptabstrahlungsrichtung (H) parallel verlaufenden Ebene planar erstreckt und eine zur Hauptabstrahlungsrichtung (H) quer verlaufende Breite (B) aufweist, wobei die Breite (b) des in Hauptabstrahlungsrichtung (H) über die Lichtabstrahlseite (9) des Halbleiterelements (3) hinausragenden Teils (31) des zweiten Abschnitts (29) des Isolierelements (5) geringer ist als die Breite (B) des ersten Abschnitts (27) des Isolierelements (5).

6. Ultra-miniaturisierte lichtemittierende Einheit (1) nach Anspruch 5, wobei die Breite (b) des in Hauptabstrahlungsrichtung (H) über die Lichtabstrahlseite (9) des Halbleiterelements (3) hinausragenden Teils (31) des zweiten Abschnitts (29) des Isolierelements (5) weniger als die Hälfte der Breite (B) des ersten Abschnitts (27) des Isolierelements (5) beträgt.

7. Ultra-miniaturisierte lichtemittierende Einheit nach einem der vorhergehenden Ansprüche, wobei sich der in Hauptabstrahlungsrichtung (H) über die Lichtabstrahlseite (9) des Halbleiterelements (3) hinausragende Teil (31) des zweiten Abschnitts (29) des Isolierelements (5) im Wesentlichen senkrecht zur Lichtabstrahlseite (9) des Halbleiterelements (3) erstreckt.

8. Ultra-miniaturisierte lichtemittierende Einheit nach einem der vorhergehenden Ansprüche, wobei der Innenleiter (15) lateral am Isolierelement (5) befestigt ist, wobei der Innenleiter (15) vom Leiterelement (25) im Isolierelement (5) elektrisch isoliert ist.

9. Ultra-miniaturisierte lichtemittierende Einheit (1) nach einem der vorhergehenden Ansprüche, wobei das Isolierelement (5) eine erste Kontaktöffnung für den ersten Kontaktbereich (49) des Leiterelements (25) und eine zweite Kontaktöffnung für den zweiten Kontaktbereich (53) des Leiterelements (25) aufweist, wobei die erste Kontaktöffnung zum Außenleiter (13) hin geöffnet ist und die zweite Kontaktöffnung eine Ausnehmung in dem in Hauptabstrahlungsrichtung (H) über die Lichtabstrahlseite (9) des Halbleiterelements (3) hinausragenden Teil des zweiten Abschnitts (29) ist.

10. Ultra-miniaturisierte lichtemittierende Einheit nach Anspruch 9, wobei der zweite Kontaktbereich (53) des Leiterelements (25) in die zweite Kontaktöffnung des Isolierelements (5) hineinragt, und wobei die Kehllötung (52) die zweite Kontaktöffnung vollständig füllt und den elektrischen Kontakt zwischen dem zweiten Kontaktbereich (53) des Leiterelements (25) und der Lichtabstrahlseite (9) des Halbleiterelements (5) herstellt.

11. Medizinisch-endoskopisches Instrument (59) mit einem distalen länglichen Einführabschnitt (63) zum minimal-invasiven Einführen in einen menschlichen oder tierischen Körper, wobei der Einführabschnitt (63) mindestens eine ultra-miniaturisierte lichtemittierende Einheit (1) nach einem der vorhergehenden Ansprüche aufweist, um damit den Körper von innen beleuchten und/oder mit Licht bestrahlen zu können.

12. Medizinisch-endoskopisches Instrument (59) nach Anspruch 11, wobei zumindest eine der mindestens einen ultra-miniaturisierten lichtemittierenden Einheit (1) an einer distalen Stirnseite (61) des Einführabschnitts (63) angeordnet ist.
